# EUROPEAN PATENT SPECIFICATION

(11) **EP 2 109 421 B1**
(45) Date of publication and mention of the grant of the patent: **13.01.2016**
(21) Application number: 08729178.7
(22) Date of filing: 06.02.2008
(51) Int. Cl.: A61F 2/38, A61F 2/30, A61F 2/46, A61B 17/17

(54) **SYSTEM FOR JOINT RESURFACE REPAIR**
SYSTEM ZUR GELENKERNEUERUNG UND -REPARATUR
SYSTÈME DE RECONSTITUTION DES SURFACES ARTICULAIRES

(30) Priority: 06.02.2007 US 888382 P
(43) Date of publication of application: 21.10.2009
(73) Proprietor: ArthroSurface, Inc., Franklin, MA 02038 (US)
(72) Inventor: EK, Steven, W., Bolton, MA 01740 (US)
(74) Representative: Kierdorf Ritschel
(86) International application number: PCT/US2008/053194
(87) International publication number: WO 2008/098061

(56) References cited:
- US-A- 4 340 978
- US-A1- 2001 010 023
- US-A1- 2002 022 889
- US-A1- 2003 100 953
- US-A1- 2006 020 343

## Description

### CROSS REFERENCE TO RELATED APPLICATIONS

This application claims the benefit of U.S. provisional patent application Serial No. 60/888,382, filed February 6, 2007. This application is also a continuation-in-part of U.S. patent application Serial No. 11/359,891, filed February 22, 2006, which itself is a continuation-in-part of U.S. patent application Serial No. 10/373,463, filed February 24, 2003, which is a continuation-in-part application of application Serial No. 10/162,533 (now U.S. Patent No. 6,679,917), filed June 4, 2002, which is itself a continuation-in-part application of application Serial No. 10/024,077 (now U.S. Patent No. 6,610,067), filed December 17, 2001, which is itself a continuation-in-part application of application Serial No. 09/846,657 (now U.S. Patent No. 6,520,964), filed May 1, 2001, which claims priority from U.S. provisional application Serial No. 60/201,049, filed May 1, 2000.

### FIELD

This disclosure relates to devices and methods for the repair of defects that occur in articular cartilage on the surface of bones, particularly the knee.

Articular cartilage, found at the ends of articulating bone in the body, is typically composed of hyaline cartilage, which has many unique properties that allow it to function effectively as a smooth and lubricious load-bearing surface. When injured, however, hyaline cartilage cells are not typically replaced by new hyaline cartilage cells. Healing is dependent upon the occurrence of bleeding from the underlying bone and formation of scar or reparative cartilage called fibrocartilage. While similar, fibrocartilage does not possess the same unique aspects of native hyaline cartilage and tends to be far less durable.

In some cases, it may be necessary or desirable to repair the damaged articular cartilage using an implant. While implants may be successfully used, the implant should have a shape substantially corresponding to the articular cartilage proximate the area where the implant is to be placed in order to maximize the patient's comfort, minimize damage to surrounding areas, and maximize the functional life of the implant.

An implant according to the preamble of claim 1 is for instance known from US 4,340,978 A. This reference discloses a so-called tricompartmental knee prosthesis which includes a femoral component providing a bearing surface for patella articulation. The bearing surface in the clockwise anterior to posterior direction is a smooth, continuous surface formed by a series of segments of surfaces of revolution, respective shapes of which are generated or defined by rotating a common generating curve around a plurality of generating axes at respective pairs of mature generating radii. The common generating curve is a smooth continuous plain curve.

The implant according to US 4,340,978 A requires a remarkable resection of bone symmetrically parallel to the femoral condyle.

Another knee joint prosthesis comprising a femoral component for implantation on a prepared femoral condyle of a knee is for instance disclosed in US 2003/0100953 A1.

US 2002/0022889 A1 also discloses a joint prosthesis which is put into place in a resection in the joint surface of a bone.

US 2001/0010023 A1 discloses a bio-absorbable cartilage repair system for regenerating damaged or destroyed articular cartilage on a joint surface of bone.

The implants as well as systems for articular surface repair according to the prior art suffer from the disadvantage that a fair amount of articular surface has to be removed in order to repair the articular.

It is thus an object of the instant application to provide an implant as well as means for insertion of such implant which allows for a minimum amount of the articular surface to be removed.

These and other objects are achieved by the implant according to claim 1 as well as by the drill guide according to claim 10.

The advantages and features of the present disclosure will become better understood with reference to the following detailed description and claims taken in conjunction with the accompanying drawings, wherein like elements are identified with like symbols, and in which:
FIG. 1 illustrates the anterior-posterior (AP) curvature 10 of a typical femoral condyle.
FIG. 2 illustrates an isometric side view of one embodiment of an implant.
FIG. 3 illustrates an isometric bottom view of the implant shown in FIG. 2.
FIG. 4 illustrates a plan side view of one embodiment of a drill guide.
FIG. 5 illustrates a perspective view of one embodiment of a measuring device.
FIG. 6 illustrates a plan side view of one embodiment of a guide pin.
FIG. 7 is a close-up of the distal end of the guide pin shown in FIG. 6.
FIG. 8 is a close-up of one embodiment of a reamer.

### DETAILED DESCRIPTION

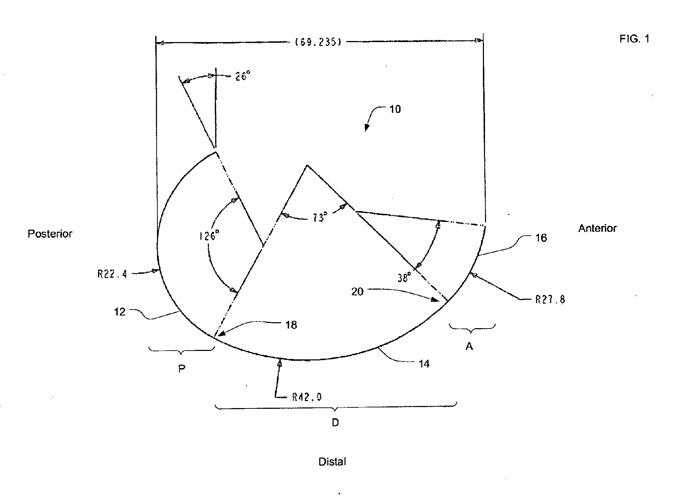

Figure 1 depicts the anterior-posterior (AP) curvature 10 of a typical femoral condyle. The curve 10 depicted in this figure may be representative of an average of AP curves from a plurality of individuals. Such curvature values may be readily found in published medical literature, for example, as may be reported in "Clinical Biomechanics 18," 2003, N. Nuno and A.M. Ahmed. As can be seen in this Figure, the AP curvature 10 of the articular surface may generally include a plurality of tangential curves having different radii of curvature.

For example, as shown in this figure, curve 10 may include a first or posterior curve 12 extending generally along the posterior region P of the femoral condyle. Curve 10 may also include a second or distal curve 14 extending generally along the distal region D of the femoral condyle), and a third or anterior curve 16 extending generally along the anterior region A of the femoral condyle. The first and second curves 12, 14 may be approximately tangential about tangent point 18 and the second and third curves 14, 16 may be approximately tangential about tangent point 20. The tangent points 18, 20 are only approximations, and the exact location of the tangent points 18, 20 may vary.

For exemplary purposes, while the AP curvature 10 may vary amongst individuals, the posterior curve 12 may have span approximately 126 degrees, the distal curve 14 may span approximately 73 degrees, and the anterior curve 16 may span approximately 38 degrees. Again, it should be noted that the extent of these curvatures may vary widely amongst individuals, and these specific ranges are provided for exemplary purposes only.

One aspect of the present disclosure is directed towards an implant that approximates at least a portion of the AP curvature 10 depicted in Figure 1. For example, for a defect that spans at least part of the distal and posterior regions of the AP curve 10, an implant provided by the present disclosure may be configured to accommodate the posterior curvature 12 and the distal curvature 14 of the femoral condyle. Advantageously, and as will be described more fully below, the implant of the present disclosure may have an AP curvature that is defined using a minimal number of data points along the AP extent of the femoral condyle. This feature may enable, for example, minimally invasive measurement procedures and implant site preparation, in accordance with previous disclosures mentioned above.

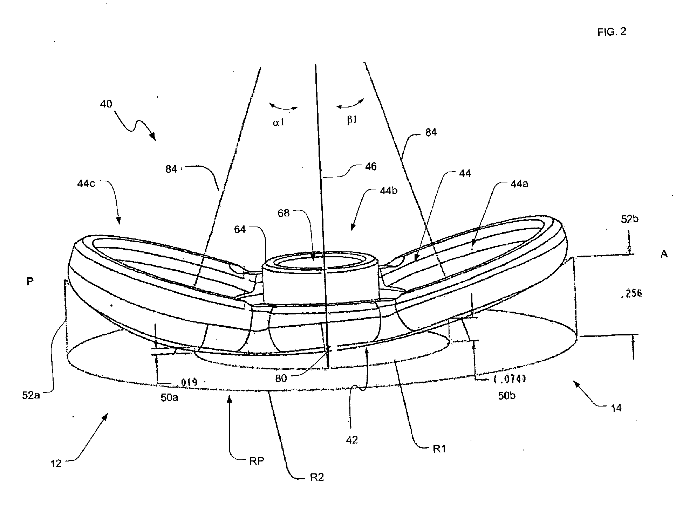

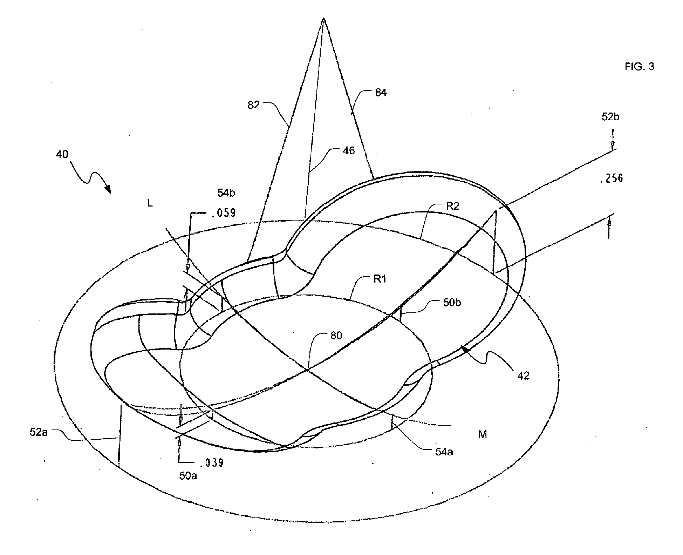

Figures 2 and 3 depict an implant according to the present invention. Figure 2 depicts an isometric side view of an implant 40. The implant 40 may include a load bearing (distal) surface 42 and a bone contacting surface 44. Surface 44 may include, for example, three or more segments 44a, 44b, 44c. In this embodiment, the implant 40 has an extended length along the AP dimension. In other words, implant 40 is generally longer in the AP dimension than the medial lateral (ML) dimension. For purposes of this example, it is assumed that the implant 40 is to be placed along a region that covers at least part of both the posterior curvature 12 and the distal curvature 14 of the femoral condyle depicted in Figure 1. However, it is equally contemplated herein that the implant 40 may be configured to define a curvature anywhere along the AP curve 10 depicted in Figure 1.

As a general statement, the curvature of the load bearing or distal surface 42 of the implant 40 may be defined by the depth from a reference plane (RP) to the load bearing surface 42 of the implant 40 at two or more different locations along the AP extent of the implant 40. In the example of Figure 2, reference plane RP is generally tangent to the load bearing surface 42 at a point 80 that passes through the reference axis 46. Reference plane RP may also be parallel to a tangential plane extending through the point 80 (e.g., the reference plane RP may be parallel to and offset a distance X from the tangent plane passing through point 80). Point 80 may be defined as a point of origin from which depth measurements may be defined. Reference axis 46 may pass through at generally the midpoint of the implant 40, i.e., through the middle of segment 44b.

According to one embodiment, at least one depth 50a and/or 50b from the reference plane RP to the surface 42 may be defined at a first distance R1 from the reference axis 46. At least one additional depth 52a and/or 52b from the reference plane RP to the surface 42 may be defined at a second distance R2 from the reference axis 46. As shown in this example, the first distance R1 is illustrated as a first circle centered about the reference axis 46 in the reference plane RP. Also as shown in this example, the second distance R2 is illustrated as a second circle centered about the reference axis 46 in the reference plane RP, where R1 < R2. Reference axis 46 may pass through at generally the midpoint of the implant 40, i.e., through the middle of segment 44b

Two or more depths, namely, at least one depth 50a and/or 50b taken at distance R1 and at least a second depth 52a and/or 52b taken at distance R2 from the reference axis 46. For example, the depth 50a may be defined as a distance between the reference plane RP and the surface 42 at a point between the reference axis 46 and the posterior (P) end of the implant 40, while depth 50b may be defined as a distance between the reference plane RP and the surface 42 at a point between the reference axis 46 and the anterior (A) end of the implant 40. Additionally, the depth 52a may be defined as a distance between the reference plane RP and the surface 42 at a point near the posterior (P) end of the implant 40, while depth 52b may be defined as a distance between the reference plane RP and the surface 42 near the anterior (A) end of the implant 40.

In one exemplary embodiment, depths 52a and 52b may be assumed approximately equal. In this case, only one depth 52a or 52b may be defined, and thus, the curvature of surface 42 of the implant 40 may be approximated using depths 52a or 52b and at least one of 50a and/or 50b. The foregoing assumes that the reference axis 46 is approximately normal to the articular surface. However, in alternative embodiments, if the reference axis is not assumed normal to the articular surface, then both depths 52a and 52b may be used to define the AP curvature of the surface 42 of the implant 40.

Each segment 44a and 44c, by virtue of the AP curvature defined by data points 50a, 50b, 52a, and/or 52b, may also have a reference axis 84 and 82, respectively. Reference axis 82 may be substantially normal to the articular surface and substantially normal to the outer surface 42 of segment 44c and passing through approximately the middle of segment 44c. Likewise, reference axis 84 may be substantially normal to the articular surface and substantially normal to the outer surface 42 of segment 44a and passing through approximately the middle of segment 44a. Since data points 52a and 52b may be approximately equal and implant 40 may be symmetrical about reference axis 46, the angle between reference axis 82 and 46, denoted as α₁ in Figure 2, and the angle between reference axis 84 and 46, denoted as β₁ in Figure 2, may therefore be approximately equal.

Thus, by defining the AP curvature of surface 42 of the implant 40 in a manner described above, the curvature of surface 42 may include two (or more) tangential, but distinct, curves of the femoral condyle. It should be noted that in most cases, the values of depth 50a and 50b may be inversely related. Thus, in a typical scenario, as the value of 50a increases, the value of 50b may decrease, and vice-versa.

As mentioned above, the implant 40 may include three or more segments 44a, 44b, 44c, wherein each segment 44a, 44b, 44c has a reference axis 82, 46, 84, respectively. The first and the third segments 44a, 44c may partially overlap the second segment 44b about opposing ends of the second segment 44b. In other words, the second segment 44b may partially overlap with each of the adjacent segments 44a and 44c. As shown, one or more of the segments 44a, 44b, 44c may include generally circular cross-sectional shape which has been truncated along the AP extent of the implant 40. In addition, any of the segments 44a, 44b, 44c may be truncated along the ML extent of the implant 40 as well.

The distal or bone facing surface 42 of the implant 40 may include one or more mounting features or fixation elements 64 for securing the implant 40 to the femoral condyle. For example, the mounting feature 64 may be configured to engage with a screw or the like (not shown) as described in U.S. Application Serial No. 10/373,463 filed February 24, 2003, U.S. Patent No. 6,679,917 issued January 20, 2004, U.S. Patent No. 6,610,067 issued August 26, 2003, U.S. Patent No. 6,520,964 issued February 18, 2003, and U.S. Provisional Application Serial No. 60/201,049 filed May 1, 2000. As shown, the mounting feature 64 may include an opening 68 (such as, but not limited to, a tapered opening) configured to engage with a corresponding post (not shown) of a screw. The opening 68 may be formed in a protrusion or the like extending generally outwardly from the bone facing surface 44. Other configurations for securing the implant 40 to the femoral condyle are also possible and contemplated herein.

As shown, the mounting feature 64 may be disposed in the second segment 44b. However, one or more mounting features 64 may be provided in the first, second, and/or third segments 44a, 44b, 44c. Optionally, the mounting feature 64 (for example, the opening 68) may be axially aligned with at least one of the axes 46, 82, 84. As shown, opening 68 of the mounting feature 64 may be axially aligned with the reference axis 46.

Figure 3 depicts an isometric bottom view of the implant 40. The AP curvature of the implant 40 may be defined as described above. The ML curvature of the implant 40 may be defined by at least two depths 54a and 54b taken at a distance from the reference axis 46. For example, depth 54a may be defined as a distance between the reference plane RP and the surface 42 at a point near the medial (M) end of the implant 40, while depth 54b may be defined as a distance between the reference plane RP and the surface 42 near the lateral (L) end of the implant 40. Based on these depths, the ML curvature of the surface 42 may be approximated as described in U.S. Application Serial No. 10/373,463 filed February 24, 2003, U.S. Patent No. 6,679,917 issued January 20, 2004, U.S. Patent No. 6,610,067 issued August 26, 2003, U.S. Patent No. 6,520,964 issued February 18, 2003, and U.S. Provisional Application Serial No. 60/201,049 filed May 1, 2000.

The system of the present disclosure may include a kit that includes a plurality of implants 40 having various AP curvatures and optionally various ML curvatures as described above. The set of implants may be based on the most common values for the AP and ML curvatures based on the most likely femoral condyle implantation sites as well as most likely values for the various depths along the AP and ML curvatures described above. For example, in such a set of implants, one or more of the AP depth values 50a, 50b, 52a, and/or 52b may vary from one implant to the next implant, for example, in ½ mm increments. In this case, an implant may be selected that most closely matches the measurements obtained from the patient's articular surface as described briefly below. Alternatively, a custom-built implant may be fabricated using these depth values.

Obtaining the depth measurements along the AP curvature and ML curvature may be obtained using the measuring tool/outrigger as described in Application Serial No. 10/373,463 filed February 24, 2003, U.S. Patent No. 6,679,917 issued January 20, 2004, U.S. Patent No. 6,610,067 issued August 26, 2003, U.S. Patent No. 6,520,964 issued February 18, 2003, and U.S. Provisional Application Serial No. 60/201,049 filed May 1, 2000.

However, in the case of the AP depth measurements, rather than using a single measuring tool, two measuring tools may be used. For example, a first measuring tool/outrigger having a radius of R1 may be used to obtain depth values 50a and/or 50b and a second measuring tool/outrigger having a radius of R2 may be used to obtain the depth values 52a and/or 52b, wherein R2 > R1. Of course, the methodology described in the aforementioned U.S. patents may be utilized using a single common reference axis (e.g., axis 46) to obtain all the measurements described herein.

The implant 40 according to the present disclosure, may be secured to the patient's articular surface as described in Application Serial No. 10/373,463 filed February 24, 2003, U.S. Patent No. 6,679,917 issued January 20, 2004, U.S. Patent No. 6,610,067 issued August 26, 2003, U.S. Patent No. 6,520,964 issued February 18, 2003, and U.S. Provisional Application Serial No. 60/201,049 filed May 1, 2000.

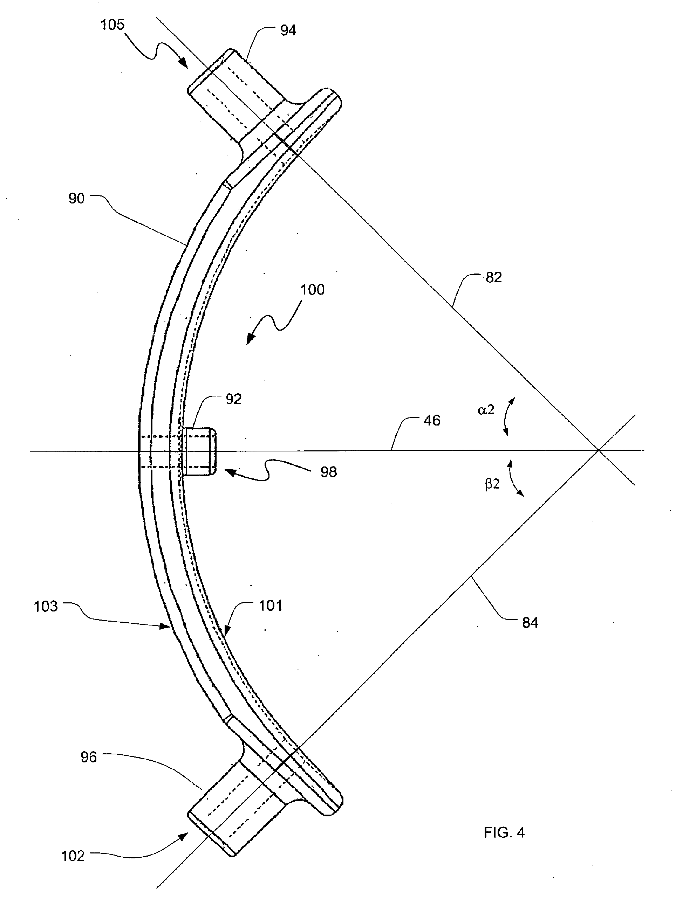

Figure 4 depicts a drill guide 100 consistent with another aspect of the present invention. Drill guide 100 may be used to establish a plurality of axes used to create a plurality of excision sites, for example, excision sites in the articular surface corresponding to segments 44a, 44b and 44c of the implant 40. The use of a multi-axes drill guide is described in U.S. Patent Application Serial No. 11/169,326 (US 2006/0020343) filed June 28, 2005. As will be explained below, the drill guide 100 may be used to establish a plurality of axes 46, 82, 84 with respect to the articular surface. Axes 46, 82 and 84 may be related to one another which may be used to guide a cutting instrument when forming the excision sites in the articular surface for the implant 40.

The drill guide 100 may be selected based on the data points obtained from the articular surface (described above). As with the implant 40 described above, the system of the present disclosure may include a kit that includes a plurality of drill guides 100 that correspond (approximately) to the data points obtained so that the angles α₂ and β₂ substantially correspond to the angles α₁ and β₂ of the implant 40. To that end, the curvature of the drill guides 100 included in such a kit will vary from one to the next, in order to establish the proper working axes 82 and 84.

Consistent with one embodiment, the drill guide 100 may generally include a body portion 90 having a generally arcuate shape in which at least a portion of the interior surface 101 of the drill guide 100 may be configured to be disposed on at least a portion of an articular surface where the implant site is to be provided. The drill guide 100 may also include a first, a second, and a third drill bushing 92, 94, and 96. The first drill bushing 92 may be configured to be disposed on the interior surface 101 of the drill guide body 90 and may extend generally radially inwardly from the drill guide 100. According to one embodiment, the first drill bushing 92 may optionally have an outer diameter that substantially corresponds to a hole drilled into the articular surface which may be used to index the drill guide 100 on the articular surface as will be discussed below. The first drill bushing 92 may also include an opening or boss 98 extending through the drill guide 100 and the first drill bushing 92. A longitudinal axis of the opening 98 in the first drill bushing 94 may substantially correspond to the reference axis 46. The first drill bushing 92 may optionally include a separate component from the body portion 90 of the drill guide 100 and may be configured to be removably coupled to the body portion 90 of the drill guide 100.

The second and third drill bushings 94, 96 may be disposed on the outer surface 103 of the drill guide body 90 and may be a spaced distance from the first drill bushing 92. The spacing of the second and third drill bushings 94, 96 may be determined based on the size of the segments 44a, 44b and 44c relative to one another, which, in turn, correspond to the excision sites in the articular surface. The measurements define the AP curve, which determines the drill guide 100 to select the angular relationship of the three axes. According to one embodiment, the second and third drill bushings 94, 96 may optionally extend generally radially outwardly from the drill guide 100. The second and third drill bushings 94, 96 may also each include an opening or boss extending through the drill guide 100 and the second and third drill bushings 94, 96. Longitudinal axes of the openings 105 and 102 may substantially correspond to the working axis 82, 84, respectively, similar to working or reference axis 46. One or more of the second and third drill bushings 94, 96 may optionally include a separate component from the body portion 90 of the drill guide 100 and may be configured to be removably coupled to the body portion 90 of the drill guide 100.

As is taught in the aforementioned published patent application, the drill guide 100 may be coordinated or indexed with the location element installed in the articular surface so that the first drill bushing 92 may be oriented coaxial with the reference axis 46 defined by the location element. For example, a guide rod may be fitted extending from the location element, and the guide rod may be received through the opening 98 in the first drill bushing 92 of the drill guide 100. According to such an embodiment, the guide rod and the location element may be provided having mating features, such as mating precision tapers. The guide rod may, therefore, be aligned along the reference axis 46. Alternatively, the drill guide 100 and the location element may include cooperating features allowing the drill guide 100 and location element to be coordinated, e.g. aligned, positioned, etc., in a predetermined manner. The first drill bushing 92 may bear against, or otherwise interact with, the location element to position the drill guide 100 at a predetermined height relative to the articular surface, based on the height of the location element relative to the articular surface.

According to a related alternative embodiment, the drill guide 100 may be indexed or positioned on the articular surface without the use of a location element. Consistent with one such embodiment, the reference axis 46 may be established, for example as described above, and a hole may be drilled into the articular surface generally along the reference axis 46. The first drill bushing 92 may be sized and shaped to be at least partially received in the hole drilled into the articular surface about the reference axis 46. The respective sizes of the hole and the first drill bushing 92 may be coordinated to achieve a predetermined tolerance and control the amount of movement, or slop, of the drill guide 100 relative to the articular surface. In one embodiment, a snug fit may be achieved between the first drill bushing 92 and the hole, thereby restricting movement of the drill guide 100 relative to the articular surface.

With the drill guide 100 located on the articular surface and indexed with the reference axis 46, the working axes 82 and 84 may be established relative to the articular surface and the reference axis 46. The working axes 82, 84 may be established by drilling reference holes into the articular surface guided by the second and third drill bushings 94 and 96. A location element may be installed into each of the reference holes created in the articular surface using the second and third drill bushings 94, 96 of the drill guide 100 as described in Application Serial No. 10/373,463 filed February 24, 2003, U.S. Patent No. 6,679,917 issued January 20, 2004, U.S. Patent No. 6,610,067 issued August 26, 2003, U.S. Patent No. 6,520,964 issued February 18, 2003, and U.S. Provisional Application Serial No. 60/201,049 filed May 1, 2000.

Once the working axes 82 and 84 and the reference axis 46 have been established, portions of the articular surface (and optionally underlying subchondral bone) may be excised to provide excision sites corresponding to segments 44a, 44b and 44c of the implant 40. According to one embodiment, the articular surface may be excised using a drill, rotating cutter, or other instrument for excising a generally circular region of the articular surface and/or subchondral bone as described in Application Serial No. 10/373,463 filed February 24, 2003, U.S. Patent No. 6,679,917 issued January 20, 2004, U.S. Patent No. 6,610,067 issued August 26, 2003, U.S. Patent No. 6,520,964 issued February 18, 2003, and U.S. Provisional Application Serial No. 60/201,049 filed May 1, 2000. Depending upon the diameter of the cutting path defined by the cutting blade or blades of the cutting instrument, at least a portion of the cutting path defined by the sweep of the cutting instrument may extend outside of the width of the condyle at the excision site.

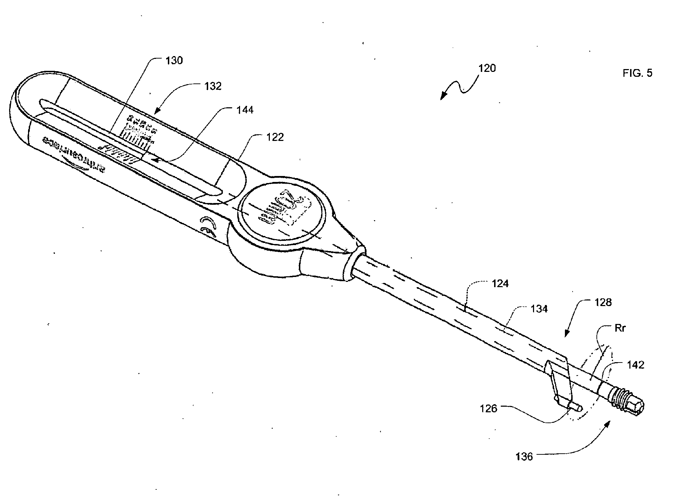

Figure 5 depicts a measuring device 120 consistent with another aspect of the present disclosure. Measuring device 120 may be used to obtain or map a plurality of points on a patient's articular surface. Instead of a separate screw element, however, the measuring device 120 of the present disclosure may include an integrally formed tap (described below) that may be readily advanced and removed into and from the bone. The measuring device 120 may comprise a housing 122 defining a longitudinally disposed passageway 124 therein. The housing 122 may comprise an outrigger 126 extending generally outwardly from the distal end 128 of the housing 122 a predefined radial distance Rr from the longitudinal axis of the housing 122. The housing 122 may also include a window or aperture 130 having a plurality of measurement markings/indicia (generally indicated by 132) for generating a measurement as will be described below.

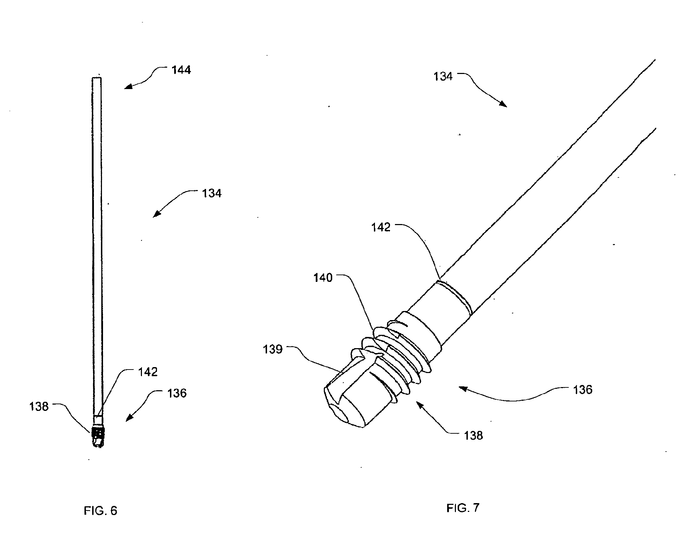

The measuring device 120 may also feature a guide pin 134, as shown in Figures 5-8, configured to be rotatably disposed within the longitudinally disposed passageway 124 of the housing 112. A distal end 136 of the guide pin 134 (best seen in Fig. 7) may include a tap 138 for boring into the articular surface. The tap 138 may include fluted regions 139 and cutting edges 140 which allow the guide pin 134 to be self drilling and self tapping.

The distal end 136 of the guide pin 134 may be inserted into the articular surface by rotating the guide pin 134. This may be preformed by hand, but may optionally include the use of a drill or the like. As the guide pin 134 is rotated, the tap 138 bores and threads a hole into the articular surface to secure the guide pin 134 relative to the articular surface. The depth which the guide pin 134 is inserted into the articular surface may be determined by one or more visual indicia located on the guide pin 134. For example, the guide pin 134 may feature an indentation or indicia (such as, but not limited to, a laser marking or the like) 142 extending generally radially inwardly a predetermined distance proximate the distal end 136 of the guide pin 134. In practice, a surgeon may screw the guide pin 134 into the articular surface until the indentation 142 is substantially coplanar with the articular surface.

Once the guide pin 134 is inserted into the articular surface proximate the region to be measured, the housing 122 of the measuring device 120 may be placed axially along the guide pin 134. The measuring device 120 may be rotated about the guide pin 134 until the outrigger 126 is proximate the area to be measured. Measurements may be obtained by contacting the outrigger 126 with the articular surface and reading which of the marking indicia 132 along the window 130 of the housing 122 are aligned with a visual indicia on the guide pin 134 (such as the proximate end 144, markings and/or indentations on the guide pin 134). This process may be repeated until the desired number of measurements are obtained.

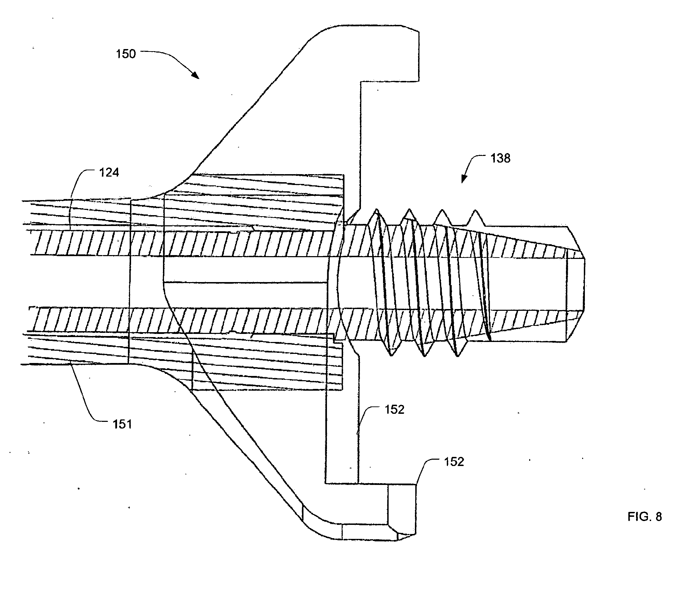

Once the desired number of measurements are taken, the housing 122 of the measuring device 120 may be removed from the guide pin 134. Using the guide pin 134 extending from the articular surface, a reamer 150, shown in Figure 8, may be axially aligned along the guide pin 134. The reamer 150 may be used to excise a region of the articular surface and may comprise a shaft 151 which may be coupled to a drill device (such as, but not limited to, a hand or power operated drill). The reamer 150 may be rotated about the guide pin 134 such that the cutting surfaces 152 of the reamer excise a portion of the articular surface.

As mentioned above, the present disclosure is not intended to be limited to a system or method which must satisfy one or more of any stated or implied object or feature of the present disclosure and should not be limited to the preferred, exemplary, or primary embodiment(s) described herein. The foregoing description of a preferred embodiment of the present invention has been presented for purposes of illustration and description. It is not intended to be exhaustive or to limit the present disclosure to the precise form disclosed. Obvious modifications or variations are possible in light of the above teachings. The embodiment was chosen and described to provide the best illustration of the principles of the present disclosure and its practical application to thereby enable one of ordinary skill in the art to utilize the present disclosure in various embodiments and with various modifications as is suited to the particular use contemplated. All such modifications and variations are within the scope of the invention, as defined by the claims.

## Claims

1. An implant (40) for replacing a portion of an articular surface of a femoral condyle comprising:
first, second, and third segments (44a, b, c), said first, said second, and said third segments (44a, b, c) comprise a bone contacting surface (44) and a load bearing surface (42), said load bearing surface (42) comprising an anterior-posterior (AP) curvature and a medial lateral (ML) curvature,
wherein said AP curvature comprises at least two tangential curves of said portion of said articular surface of said femoral condyle, said tangential curves having different radii of curvature,
**characterized in that**
said second and said third segments (44b, c) partially overlap said first segment (44a) on opposite ends of said first segment (44a),
and wherein said first, said second, and said third segments (44a, b, c) each comprise a truncated, generally circular cross-sectional shape.

2. The implant (40) of claim 1, wherein said first, said second, and said third segments (44a, b, c) each comprise a generally circular shape truncated along said AP curvature of said implant.

3. The implant (40) of claim 2, wherein said first, said second, and said third segments (44a, b, c) each comprise a generally circular shape truncated along said ML curvature of said implant.

4. The implant (40) of claim 1, wherein said bone contacting surface comprises at least one mounting feature configured to secure said implant to said femoral condyle.

5. The implant (40) of claim 4, wherein said at least one mounting feature is configured to securely engage with a mounting screw.

6. The implant (40) of claim 5, wherein said at least one mounting feature includes an opening configured to engage with a post of said mounting screw.

7. The implant (40) of claim 1, wherein said first, said second, and said third truncated, generally circular segments comprise first, second, and third axes (82, 46, 84)extending through respective centers of said segments, respectively, wherein said first axis is substantially normal to said portion of said articular surface.

8. The implant of claim 7, wherein said second and said third axes (46, 84) are substantially normal to said portion of said articular surface.

9. The implant of claim 8, wherein a first angle α between said first axis (82) and said second axis (46) and a second angle β between said first axis (82) and said third axis (84) are substantially symmetrical.

10. A drill guide (100) for establishing a plurality of axes (46, 82, 84) in an articular surface and adapted to create a plurality of circular excision sites in the articular surface corresponding to segments (44a, b, c) of the implant (40) according to anyone of the preceding claims, said drill guide comprising:
a body portion (90) comprising a generally arcuate shaped interior surface, wherein at least a portion of said interior surface is configured to be disposed on at least a portion of said articular surface; and
first, second and third bushings (92, 94, 96) spaced along a length of said body portion (90) and configured to establish first, second and third axes (46, 82, 84), respectively, wherein each of said first, said second and said third axes (92, 94, 96) are configured to be substantially normal to said articular surface at three different points, respectively, along a curvature of said articular surface comprising at least two tangential curves, said tangential curves having different radii of curvature.

11. The drill guide of claim 10, wherein at least one of said first, said second and said third bushings (92, 94, 96) are removably coupled to said body portion (90).

12. The drill guide of claim 10, wherein said second bushing is disposed along said body portion between said first and said third bushings, and wherein a first angle α between said first axis and said second axis and a second angle β between said second axis and said third axis are substantially symmetrical.

13. The drill guide of claim 10, wherein said second bushing (94) extends generally outwardly from said interior surface of said body portion.

14. The drill guide of claim 13, wherein said second bushing (94) comprises an outer diameter substantially corresponding to a diameter of hole formed in said articular surface.

## Patentansprüche

1. Implantat (40) zum Ersetzen eines Teils einer Gelenkfläche eines Oberschenkelkondylus, umfassend: erste, zweite und dritte Segmente (44a, b, c), wobei die ersten, zweiten und dritten Segmente (44a, b, c) eine Knochenkontaktoberfläche (44) und eine Lastaufnahmeoberfläche (42) umfassen, wobei die Lastaufnahmeoberfläche (42) eine Anterior-Posterior(AP)-Krümmung und eine Medial-Lateral(ML)-Krümmung aufweist, wobei die AP-Krümmung mindestens zwei tangentiale Kurven des Teils der Gelenkfläche des Oberschenkelkondylus umfasst, wobei die tangentialen Kurven unterschiedliche Krümmungsradien aufweisen, **dadurch gekennzeichnet, dass** die zweiten und dritten Segmente (44b, c) das erste Segment (44a) auf gegenüberliegenden Enden des ersten Segments (44a) teilweise überlappen und wobei die ersten, zweiten und dritten Segmente (44a, b, d) jeweils einen kegelstumpfförmigen kreisförmigen Querschnitt aufweisen.

2. Implantat (40) nach Anspruch 1, bei welchem die ersten, zweiten und dritten Segmente (44a, b, c) jeweils eine kreisförmige kegelstumpfförmige Form entlang der AP-Krümmung des Implantats aufweisen.

3. Implantat (40) nach Anspruch 2, bei welchem die ersten, zweiten und dritten Segmente (44a, b, c) jeweils eine kreisförmige kegelstumpfförmige Form entlang der ML-Krümmung des Implantats aufweisen.

4. Implantat (40) nach Anspruch 1, bei welchem die Knochenkontaktfläche wenigstens ein Befestigungsmittel aufweist, das zur Sicherung des Implantats an dem Oberschenkelkondylus ausgebildet ist.

5. Implantat (40) nach Anspruch 40, bei welchem wenigstens ein Befestigungsmittel zum sicheren Eingriff mit einer Befestigungsschraube ausgebildet ist.

6. Implantat (40) nach Anspruch 5, bei welchem das wenigstens eine Befestigungsmittel wenigstens eine Öffnung aufweist, die so ausgebildet ist, dass sie mit einem Schaft einer Befestigungsschraube zusammenwirkt.

7. Implantat (40) nach Anspruch 1, bei welchem die ersten, zweiten und dritten kegelstumpfförmigen zylindrischen Segmente erste, zweite und dritte Achsen (82, 46, 84) aufweisen, die sich jeweils durch das Zentrum der Segmente erstrecken, wobei sich die erste Achse im Wesentlichen normal zu dem Teil der Gelenkfläche erstreckt.

8. Implantat (40) nach Anspruch 7, bei welchem die zweiten und dritten Achsen (46, 84) sich im Wesentlichen normal zu dem Teil der Gelenkfläche erstrecken.

9. Implantat (40) nach Anspruch 8, bei welchem der Winkel a zwischen der ersten Achse 82 und der zweiten Achse 46 und ein zweiter Winkel β zwischen der ersten Achsen 82 und der dritten Achse 84 im Wesentlichen symmetrisch sind.

10. Bohrführung (100) zur Herstellung einer Vielzahl von Achsen (46,82, 84) in einer Gelenkfläche, die ausgebildet ist, um eine Vielzahl von kreisförmigen Ektomien in der Gelenkfläche entsprechend den Segmenten 44a, b, c des Implantats (40) gemäß einem der vorhergehenden Ansprüche herzustellen, wobei die Bohrführung folgendes umfasst:
einen Körper (90), der eine bogenförmige innere Oberfläche aufweist, wobei wenigstens ein Teil der inneren Oberfläche so ausgebildet ist, dass sie auf wenigstens einem Teil der Gelenkfläche angeordnet werden kann; und erste, zweite und dritte Buchsen (92, 94, 96), die sich entlang einer Länge des Körpers (90) erstrecken und jeweils zur Einrichtung erster, zweiter und dritter Achsen (86, 82, 84) ausgebildet sind, wobei jede der ersten, zweiten und dritten Achsen (92, 94, 96) so ausgebildet ist, dass sie sich im Wesentlichen normal zu der Gelenkfläche jeweils an drei verschiedenen Punkten entlang einer Krümmung der Gelenkfläche erstreckt, wobei die Gelenkfläche wenigstens zwei tangentiale Kurven aufweist, die verschiedene Krümmungsradien aufweisen.

11. Bohrführung nach Anspruch 10, bei welchem wenigstens einer der ersten, zweiten und dritten Buchen (92, 4, 96) lösbar mit dem Körper (90) verbunden ist.

12. Bohrführung nach Anspruch 10, wobei die zweite Buchse entlang des Körpers zwischen der ersten und der dritten Buchse angeordnet ist und wobei ein erster Winkel a zwischen der ersten Achse und der zweiten Achse und ein zweiter Winkel β zwischen der zweiten Achse und der dritten Achse im Wesentlichen symmetrisch sind.

13. Bohrführung nach Anspruch 10, bei welcher die zweite Buchse (94) sich auswärts von der inneren Oberfläche des Körpers erstreckt.

14. Bohrführung nach Anspruch 13, wobei die zweite Buchse (94) einen Außendurchmesser umfasst, der im Wesentlichen dem Durchmesser des in der Gelenkfläche gebildeten Lochs entspricht.

## Revendications

1. Implant (40) pour remplacer une partie de la surface articulaire d'un condyle fémoral comprenant :
des premier, deuxième et troisième segments (44a, b, c), ledit premier, ledit deuxième et ledit troisième segment (44a, b, c) comprenant une surface (44) de contact avec l'os et une surface portante (42), ladite surface portante (42) présentant une courbure antéropostérieure (AP) et une courbure médio-latérale (ML),
dans lequel ladite courbure AP comprend au moins deux courbes tangentielles de ladite partie de ladite surface articulaire dudit condyle fémoral, lesdites courbes tangentielles ayant des rayons de courbure différents,
**caractérisé en ce que** ledit deuxième et ledit troisième segment (44b, c) chevauchent partiellement ledit premier segment (44a) sur les extrémités opposées dudit premier segment (44a), et
dans lequel ledit premier, ledit deuxième et ledit troisième segment (44a, b, c) présentent chacun une forme transversale dans l'ensemble circulaire, tronquée.

2. Implant (40) selon la revendication 1, dans lequel ledit premier, ledit deuxième et ledit troisième segment (44a, b, c) présentent chacun une forme dans l'ensemble circulaire, tronquée suivant ladite courbure AP dudit implant.

3. Implant (40) selon la revendication 2, dans lequel ledit premier, ledit deuxième et ledit troisième segment (44a, b, c) présentent chacun une forme dans l'ensemble circulaire, tronquée suivant ladite courbure ML dudit implant.

4. Implant (40) selon la revendication 1, dans lequel ladite surface de contact avec l'os comprend au moins un élément de montage configuré pour bien fixer ledit implant audit condyle fémoral.

5. Implant (40) selon la revendication 4, dans lequel ledit élément de montage est configuré pour coopérer solidement avec une vis de montage.

6. Implant (40) selon la revendication 5, dans lequel ledit élément de montage comprend une ouverture configurée pour coopérer avec un tenon de ladite vis de montage.

7. Implant (40) selon la revendication 1, dans lequel dans lequel ledit premier, ledit deuxième et ledit troisième segment dans l'ensemble circulaires, tronqués comprennent un premier, un deuxième et un troisième axe (82, 46, 84) qui passent par les centres respectifs desdits segments, respectivement, dans lequel ledit premier axe est sensiblement perpendiculaire à ladite partie de ladite surface articulaire.

8. Implant selon la revendication 7, dans lequel ledit deuxième et ledit troisième axe (46, 84) sont sensiblement perpendiculaires à ladite partie de ladite surface articulaire.

9. Implant selon la revendication 8, dans lequel un premier angle α entre ledit premier axe (82) et ledit deuxième axe (46) et un second angle β entre ledit premier axe (82) et ledit troisième axe (84) sont sensiblement symétriques.

10. Guide-foret (100) pour établir une pluralité d'axes (46, 82, 84) dans une surface articulaire et apte à créer dans la surface articulaire une pluralité de sites circulaires d'excision correspondant aux segments (44a, b, c) de l'implant (40) selon l'une quelconque des revendications précédentes, ledit guide-foret comprenant :
une partie de corps (90) comprenant une surface intérieure de forme dans l'ensemble arquée, dans laquelle au moins une partie de ladite surface intérieure est configurée pour être disposée sur au moins une partie de ladite surface articulaire ; et
des première, deuxième et troisième douilles (92, 94, 96) espacées sur la longueur de ladite partie de corps (90) et configurées pour établir respectivement les premier, deuxième et troisième axes (46, 82, 84), dans lequel ledit premier, ledit deuxième et ledit troisième axe (92, 94, 96) sont chacun configurés pour être sensiblement perpendiculaires à ladite surface articulaire respectivement en trois points différents, le long d'une courbure de ladite surface articulaire comprenant au moins deux courbes tangentielles, lesdites courbes tangentielles ayant des rayons de courbure différents.

11. Guide-foret selon la revendication 10, dans lequel au moins une desdites première, deuxième et troisième douilles (92, 94, 96) est accouplée amovible à ladite partie de corps (90).

12. Guide-foret selon la revendication 10, dans lequel ladite deuxième douille est disposée le long de ladite portion de corps entre ladite première et ladite troisième douilles et dans lequel un premier angle α entre ledit premier axe et ledit deuxième axe et un second angle β entre ledit deuxième axe et ledit troisième axe sont sensiblement symétriques.

13. Guide-foret selon la revendication 10, dans lequel ladite deuxième douille (94) s'étend dans l'ensemble vers l'extérieur depuis ladite surface intérieure de ladite partie de corps.

14. Guide-foret selon la revendication 13, dans lequel ladite deuxième douille (94) présente un diamètre extérieur correspondant sensiblement à un diamètre de trou pratiqué dans ladite surface articulaire.
